Europäisches Patentamt

European Patent Office

Office européen des brevets

(19)

(11) EP 0 664 886 B1

(12) **EUROPEAN PATENT SPECIFICATION**

(45) Date of publication and mention
of the grant of the patent:
**02.08.2000 Bulletin 2000/31**

(51) Int. Cl.[7]: **G01N 33/543**, G01N 33/558,
G01N 33/577, G01N 33/569

(21) Application number: **93923331.8**

(22) Date of filing: **07.10.1993**

(86) International application number:
**PCT/US93/09646**

(87) International publication number:
**WO 94/09367 (28.04.1994 Gazette 1994/10)**

(54) **ALLOANTIGEN ENHANCEMENT ASSAY**

ALLOANTIGEN-VERSTÄRKUNGSTEST

ESSAI AMELIORE A ALLOANTIGENES

(84) Designated Contracting States:
**DE FR GB**

(30) Priority: **16.10.1992 US 961579**

(43) Date of publication of application:
**02.08.1995 Bulletin 1995/31**

(73) Proprietor:
**SANGSTAT MEDICAL CORPORATION
Fremont, California 94555 (US)**

(72) Inventors:
• **POULETTY, Philippe
Atherton, CA 94025 (US)**
• **CHIN-HAI, Chang
Los Altos, CA 94024 (US)**

(74) Representative:
**O'Brien, Caroline Jane et al
MEWBURN ELLIS
York House
23 Kingsway
London WC2B 6HP (GB)**

(56) References cited:
**WO-A-93/04372          US-A- 5 059 524**

**Description**

INTRODUCTION

Technical Field

The technical field of this invention is HLA typing.

Background

There is substantial interest in being able to type human leukocyte antigens (HLAs) for a variety of reasons. In many situations it has been found that specific HLA alleles may be associated with a susceptibility to a particular disease. For example, HLA-B27 has been associated with ankylosing spondylitis and related diseases. When transplanting organs to a host it is desirable that the organs be matched, so as to minimize the risk of rejection. HLA typing may also find application is determining lineage, epidemiology and the like.

There is an extensive family of HLA antigens divided into Class I and Class II. In each of the classes, there are polymorphic regions. These sites may or may not provide for epitopes which will induce an immune response which will allow for the preparation of antisera or monoclonal antibodies which are specific for a specific HLA allele and able to distinguish that HLA allele from other HLA allele.

This situation is exemplified by the cross-reactivity between HLA-B27 and HLA-B7 where monoclonal antibodies are not readily available which are specific for HLA-B27, so as not to cross-react with HLA-B7 or other HLA allele.

Since mammals are diploid, there will be pairs of HLA antigens as to each of the particular groups. Thus, unless one can determine specifically a particular HLA allele, one cannot be certain whether there are two different alleles or one is observing cross-reactivity. There is, therefore, substantial interest in developing methods which will allow for the accurate detection of a particular HLA allele, where substantial cross-reactivity is observed with other HLA alleles.

Relevant Literature

Sakaguchi, *et al*. (1988) *Human Immunology* 21, 193-207 describes the use of monoclonal antibodies in determination of HLA-B27 and a double determinant immunoassay for detection of HLA-B27. Villar, *et al*. (1989) *Eur. J. Immunol*. 19, 1835-1839 describe the detection of Class I molecules from a variety of sources. Toxiadis and Grosse-Wilde, (1989) Vox Sang, 56, 196-199 describe the detection of HLA Class I proteins. Ferreira, *et al*. (1988) *Clin. Chim. Acta*. 174, 207-211 describe the use of a solid-phase enzyme immunoassay for detection of HLA Class I antigens in sera.

WO 93/04372 and US 5059524 are patent publications disclosing typing methods. WO 93/04372 is a document falling within the terms of Article 54(3) of the European Patent Convention.

SUMMARY OF THE INVENTION

Methods and compositions are provided for detecting closely-related alleles employing immunoassays, where there is no available antibody to distinguish between two alleles. The methods and compositions find specific exemplification in relation to distinguishing the HLA alleles B27 and B7, where B27 is of diagnostic interest. The method employs an antibody cross-reactive with the two alleles bound to a surface, an antibody specific for one of the two alleles, but not the other, and a receptor for a conserved region of the two alleles, which receptor is conjugated to a label for detection. The method employs negative and positive controls to provide for detection of a minimal signal and an enhanced signal.

DESCRIPTION OF THE SPECIFIC EMBODIMENTS

Methods and compositions are provided for the accurate detection and quantitation of one of two polymorphic antigens in a sample, where the antigens are characterized by having no useful receptor readily available to distinguish between the two antigens, the antigens are members of a much larger group of antigens, usually substantially in excess of 10, where there is substantial cross-reactivity between the antigens in sharing numerous epitopes, and differences between the antigens may be subtle, involving only one or a few amino acids.

The method involves having an antibody which is able to bind to the two cross-reactive soluble alleles, exemplified by HLA-B27, and -B7, where there is no antibody which can specifically distinguish B27 from B7; an antibody specific for the cross-reactive antigen which is not of interest, exemplified by B7; a positive control comprising B7; a negative control comprising buffer; and a conjugate which recognizes a conserved region for the two alleles or common epitope, where the conjugate comprises a label which allows for detection.

The subject method has particular application for physiological samples, particularly blood or fractions thereof, *e.g.*, serum and plasma, ("blood" intends blood and fractions thereof), more particularly associated with HLA antigens, particularly soluble antigens which may be found in blood. The sample may be subject to prior treatment, such as removal of cells in the case of blood to provide plasma or serum, dilution with buffer, or other treatment as appropriate. Conveniently, blood or serum may be used. The volume of the sample used will usually be about 5-250 μl usually 10-100 μl.

An antibody cross-reactive with the two alleles is bound to a support, which will usually result in binding of the detectable conjugate to the support, in the presence of the alleles. The bound antibody may be sufficient to bind all, substantially all, or greater than about 50%, of the cross-reacting alleles present in the sample aliquot used in the assay, based on the amount encountered in normal samples. The antibody may or may not have equal affinity for the two alleles, preferably having a higher affinity for the allele of interest, preferably at least about a 25% higher affinity. In addition, either antisera or monoclonal antibody may be used, using antisera when available form multiparous women, or monoclonal antibody, as appropriate. Thus, where one wishes to determine the presence of B27, one would have an antibody which is cross-reactive with B27 and B7, for which no convenient antibody is available which can distinguish between the two. The antibody may be bound to the surface in any convenient manner, either covalently or non-covalently, so long as it is maintained bound to the support during the course of the assay. Methods for binding antibodies to supports are well-known in the literature and need not be described here.

Any convenient support may be used, such as microtiter wells, slides, tubes, *etc.*, where each of the individual determinations can be maintained independent of the other determinations and the label can be discretely determined.

In carrying out the determination, normally both a positive and negative control will be employed. The positive control will comprise a predetermined amount of HLA-B7, generally at a concentration in the range of about 10ng/ml to 5μg/ml, more usually in the range of about 50ng/ml to 1 μg/ml. The positive control provides a base value for the presence of HLA-B7 and an enhanced value, which will be described below. The negative control is buffer or any other medium, such as human serum, which should not affect the value obtained in relation to the presence of HLA-B7 or -B27.

In order to distinguish between the possibilities of there being neither B7 nor B27, there is only B7 or B27, or there is both B7 and B27, anti-B7 is added to the sample and to some controls, but not others. For the most part, the bound antibody will not be limiting as to the binding of the HLA to the surface. The anti-B7/B27 antibody will normally have greater affinity for the allele of interest, B27, as compared to the allele not of interest, so that when B7 or B27 is in the sample, one will usually get a greater signal with B27. However, when B7 is present and one adds the enhancing anti-B7, the observed signal will substantially increase. Since one may use different sized aliquots of sample in the analyte determination as compared to the controls, observed values will be adjusted accordingly. The amount of B7 which is added will generally range from about 100ng/ml to 200μg/ml, more usually 1 μg/ml to 50μg/ml, based on a sample volume of about 100 μl to 1 ml.

In order to determine the presence of B7 and/or B27, a conjugate is used which binds to a consensus sequence or public epitope of the two alleles. In the case of Class I HLA, conveniently, an antibody for β$_2$-microglobulin may be employed. Alternatively, one may use an antibody for the constant region of the α chain or other polyallele sequence. While antibodies, particularly monoclonal antibodies, are the most convenient, any other receptor which binds specifically to Class I HLA may also be used. The volume of the conjugate solution will generally range from about 10-500 μl.

The label may be any convenient label, which provides the desired degree of sensitivity. Commonly employed labels include enzymes, fluorescers, radioisotopes, and the like. Of particular interest are enzymes, where the enzyme has a substrate which provides for a colored product, particularly a colored product which can be readily detected in a spectrophotometer. The enzyme substrate solution may be varied widely, generally being the range of about 20-500 μl.

The assay medium will generally be buffered at a pH in the range of about 6-9. Various buffers may be employed, such as phosphate, Tris, MOPS, HEPES, and the like. The concentration of the buffer will generally be sufficient to maintain the desired pH, generally being from about 10 mM to about 0.5 M.

In carrying out the assay, conveniently a container having a plurality of wells may be employed, where the wells are positioned in parallel rows. A device which fulfills the requirements of the subject determinations is a microtiter plate. As a first step, one adds a predetermined volume of the anti-B7 solution to a first set of rows, for discussion purposes, the even rows. One then adds to individual wells in the odd rows the negative calibrator and the positive calibrator, desirably having three wells for the negative calibrator and three wells for the positive calibrator, so that the values can be averaged. To the first set of even wells, the volume of the negative and the positive calibrators added will be less than the volume used for the second set of odd rows, the total volume of the anti-B7 medium and the calibrator mediums in each well being equal to the total volume of the negative calibrator and positive calibrator solutions added to wells in the second odd set of rows. To the remaining wells, both odd and even, sample is added in an amount so as to provide the same total volume in each well. The total volume will generally be in the range of about 25-500 μL, preferably in the range of about 50-200 μL, more particularly 100 μL. Thus, each of the wells will have the same volume as a result of combining the anti-B7 solution with calibrators or sample, or as the result of adding solely the calibrators or sample.

The assay mixtures are then incubated for sufficient time to ensure that reaction can occur between the various binding members. Desirably, the incubation will be at least about 30 min and not more than about 6 h, generally running from about 1 h to 3 h. The incubation may be at any convenient temperature, room temperature sufficing, and being preferred because of the convenience.

After completion of the incubation, each of the wells will be thoroughly washed, to ensure that there is substantially no non-specific binding of HLAs to the wells. The washing may be a single washing, but will normally be a repetitive washing of at least two times and not more than about eight times, it being found that about 3-6 times suffices, where the wash solution is at least about equal to the original volume of the assay mixture and not more than about 5 times the original volume of the assay mixture for each washing.

After completion of the washing, the conjugate is then added to each well, where the volume of the conjugate will generally be in the range of about 25-200 μl at a concentration of about 100ng/ml to 10μg/ml. The assay mixtures are then incubated for a second time, usually at least about 0.5 h and not more than about 3 h, generally from about 1-2 h. The individual assay mixtures are then washed a second time, substantially in the manner described previously, to ensure that is substantially no non-specific binding of the conjugate. After completion of the washing, a substrate solution is added to each well, generally providing a volume of about 25-200 μl. The mixture is then incubated for sufficient time for reaction to occur to provide for detectable signal. Usually, about 5-100 min will suffice, generally from about 20-60 min will suffice. At the end of the reaction time, the reaction is stopped by any convenient means, such as an enzyme inhibitor, a denaturant, or the like. Illustrative stop solutions include 1N HCl, $H_2SO_4$, $H_3PO_4$, or the like. Normally the inhibition of further reaction will be a solution, which will have a volume which does not unduly dilute the substrate solution, usually being not more than twice the volume of the substrate solution and not less than about 0.25 times the volume of the substrate solution. Each of the wells may then be read in accordance with the absorption spectrum of the product.

Where an enzyme is not employed as the label, the steps may be varied, since there will be no need to add a substrate solution. For example, with a fluorescer or radioisotope, after washing the assay mixture free of non-specifically bound conjugate, one may then read the fluorescence or radioactivity in accordance with conventional ways.

To determine whether B27 is present, one may average the readings for the negative and positive controls. One then calculates the *per cent* enhancement for the positive control in each specimen by dividing the result obtained in the presence of anti-B7 by the result obtained in the absence of B7 times 100. One then calculates two values, arbitrarily designated as DV1 and DV2, where DV1 is a function of the negative control, such as to provide a cutoff value distinguishing between reactive and non-reactive samples, and where DV1 is, for example, the OD of untreated negative control plus a percentage of the OD of the untreated positive control and DV2 is a function of the percentage enhancement of the positive control. The particular values will be dependent on the particular format of the assay. For the assay described in the experimental section, DV1 is $2 \times$ OD of untreated negative control plus 20% OD of untreated positive control and DV2 is $0.8 \times$ *per cent* enhancement for positive control. The presence of HLA-B27 is affirmed when the OD of a sample without anti-B7 is equal to or greater than DV1 and when its percentage enhancement is less than DV2.

Kits can be provided which comprise containers, *e.g.,* microtiter plates, where the container walls are coated with the cross-reacting monoclonal antibody, an enhancing monoclonal antibody to one of the alleles, and a monoclonal antibody-label conjugate which binds to a public or common epitope of the two alleles. Optionally, one of the alleles may be provided for a control, particularly the allele to which the enhancing monoclonal antibody binds. For an enzyme label, substrate could also be included as well as stopping solution. Also, buffer may be included.

The following examples are offered by way illustration and not by way limitation.

EXPERIMENTAL

A. Reagents.

Positive calibrator (PC): Human serum or plasma reactive in the ELISA assay, 0.01% thimerosal. Lyophilized (3 ml).

Negative calibrator (NC): Human serum or defibrinated plasma unreactive in the ELISA assay, 0.01% thimerosal, lyophilized [3ml reconstituted].

B27 microtiter well strips: Microassay 96-well plate (Nunc) coated with murine monoclonal antibodies to HLA B7/B27 antigen [KS4].

Enhancing solution (ES): Aqueous solution of murine monoclonal to a human HLA-B7 antigen [MB-40] in PBS containing protein stabilizers and 0.01% thimerosal.

B27 conjugate: Commercially-available horseradish peroxidase labeled rabbit anti-human $\beta_2$-microglobulin, diluted in a diluent.

OPD tablet: 30 mg o-phenylene diamine dihydrochloride.

Substrate buffer: Aqueous solution of 0.05 M phosphate-citrate buffer pH 5.0 containing 0.1% $H_2O_2$, 0.01% thimerosal.

Wash solution (10X): Aqueous solution containing 0.1 M phosphate, 0.05% TWEEN-20$^®$ and 0.01% thimerosal.

Stop solution: 1 N $H_2SO_4$.

Deionized water containing 0.01% thimerosal is used for reconstitution.

Note: Reconstitute PC with 1.5 ml water prior to use. If crystals form in the 10X wash solution, warm up in 37°C incubator to dissolve it before use.

B. Protocol.

1. Add 80 µl of the enhancing solution (ES) to all wells of the odd-numbered rows in the microtiter plate. Leave all wells of the even-numbered rows empty.

2. To the first odd-numbered row add 20 µl of negative calibrator (NC) to the first three wells and 20 µl of positive calibrator (PC) to the second three wells. Add 20 µl of each sample into one well for the remaining wells of all odd-numbered rows.

3. Immediately add 100 µl of NC and PC in triplicate to the corresponding wells in the first even-numbered row. Add 100 µl of each sample to the corresponding single well in all even-numbered rows. Incubate the plate at room temperature for 2 h. Begin timing after the last well has been added. Cover the plate to minimize evaporation loss.

4. Dilute appropriate amount of the 10X wash solution to 1X with deionized water and assemble it into the ELISA plate wash station (such as Bio-Tek EL-403 Auto Washer). Wash the plate 5 times with 325 µl of wash solution each time. If washing manually, fill wells to the top with wash solution for each cycle and remove the solution by aspiration or dumping into the sink.

5. Add 100 µl of B27 conjugate to each well. Cover the plate and incubate at room temperature for 90 min.

6. Prepare the Substrate Solution: At about 5 min before the end of step 5 add one OPD tablet to 10 ml of the substrate buffer. Note: Substrate solution must be used within 15 min of preparation.

7. Wash the plate as in step 4.

8. Add 100 µl of the substrate solution prepared in step 6 to each well and incubate at room temperature for 30 min. Keep the plate covered in the dark.

9. Add 100 µl of stop solution to each well. The sequence and rate of addition of the stop solution must match the sequence and rate of addition of substrate in step 8.

10. Read the absorbance of each well with an ELISA reader at a wave length of 490-498 nm within 5-8 min of stopping the reaction. A reference wavelength of 600-650 nm may also be used.

C. Interpretation of Assay Results.

1. Average the triplicate readings of NC and PC.

2. Calculate the *per cent* enhancement for PC and each specimen:

$$\text{\% Enhancement} = (\text{MB40 - treated OD / untreated OD}) \times 100$$

3. Calculate:

$$DV1 = \text{OD of untreated NC} + 20\% \text{ OD of untreated PC}$$

$$DV2 = 0.8 \times \text{\% enhancement for PC}$$

4. For the specimen to be HLA-B27 positive it must meet the following requirements:

(a) either OD of untreated specimen is $\geq 2$; or

(b) both of the following:

    i. untreated OD $\geq$ DV1, and

    ii. % enhancement $\leq$ DV2.

If % enhancement is between DV2 and 1.5 x DV2, the result is indeterminate; and if the result is different from one of the above (untreated OD <DV1 or enhancement > DV2], the specimen is HLA-B27 negative.

Following the above procedure, the following results were obtained.

Table 1

| Comparison of sHLA-STAT™ B27 with microlymphocytotoxicity. | | | |
| --- | --- | --- | --- |
| Overall In-House Study: 685 Specimens from 601 Individuals | | | |
| | sHLA-STAT™ B27 ELISA | | |
| | **B27+** | **B27-** | **IND**** |
| **HLA PHENOTYPE*** | | | |
| B27+ | 154 | 16 | 8 |
| B27- | 5 | 482 | 20 |

*Microlymphocytotoxicity
Sensitivity: 90.59%
Agreement: 96.8%
Specificity: 98.2%
**Indeterminate: 4.09%

It is evident from the above results, that the subject method provides a convenient, accurate determination of the presence of an allele, which cannot be specifically bound by an antibody due to cross-reactivity with a second allele. As exemplified with HLA-B7 and B27, the subject method substantially avoids false results, while giving some quantitative and objective indication of the amount of the allele of interest present. The reagents employed are safe, common to many other assays, so that technicians are familiar with the reagents, and can be readily run and the determination made with inexpensive equipment.

## Claims

1.  A method for determining the presence of a soluble first HLA antigen in a sample with antibody which cross-reacts with said first and a second HLA antigen, said method comprising:

    combining aliquots of said sample with said cross-reacting antibody in first and second containers in the absence and presence of anti-second HLA antigen, respectively, wherein said cross-reacting antibody is bound to the surface of said containers;
    combining a separate negative control lacking said first and second HLA antigens with said cross-reacting antibody and a separate positive control comprising said second HLA antigen with said cross-reacting antibody in the absence and presence of anti-second HLA antigen, respectively, wherein the presence of anti-second HLA antigen acts to enhance the signal;
    adding a conjugate of antibody which binds to a common epitope of said first and second HLA antigens at other than the binding site of said cross-reacting antibody and a detectable label; and
    determining a signal from said detectable label in said first and second containers and said negative and positive controls; wherein cut-off values based on said positive and negative controls are used to determine whether said sample comprises said first HLA antigen.

2.  A method according to claim 1, wherein negative and positive controls are performed in conjunction with said determining.

3. A method for determining the presence of soluble HLA-B27 in a sample with cross-reacting antibody which cross-reacts with HLA-B7 and -B27, said method comprising:

combining aliquots of said sample with said cross-reacting antibody in first and second containers in the presence and absence of anti-HLA-B7, respectively, wherein said cross-reacting antibody is bound to the surface of said containers;

combining a separate negative control lacking HLA-B27 and HLA-B7 antigens with said cross-reacting antibody and a separate positive control comprising HLA-B7 antigen with said cross-reacting antibody in the absence and presence of anti-HLA-B7 antigen, respectively, wherein the presence of anti-HLA-B7 antigen acts to enhance the signal;

adding a conjugate of antibody which binds to a common epitope of HLA-B27 and HLA-B7 at other than the binding site of said cross-reacting antibody and a detectable label; and

determining a signal from said detectable label in said first and second containers and said negative and positive controls;

wherein cut-off values based on said positive and negative controls are used to determine whether said sample comprises HLA-B27 antigen.

4. A method according to claims 1 or 3, wherein said sample is a blood sample.

5. A method according to claim 3, wherein said cross-reacting antibody is a monoclonal antibody having a greater affinity for HLA-B27 than HLA-B7.

6. A method according to claims 1 or 3, wherein a plurality of samples are tested using microtiter plates.

7. A method according to claims 1 or 3, wherein said label is an enzyme and including the additional steps of:

adding enzyme substrate to said containers;
incubating for sufficient time to produce a signal and stopping the enzyme reaction.

8. A kit comprising (i) a plurality of containers coated with antibody cross-reactive with two HLA antigens, (ii) antibody specific for only one of said HLA antigens, (iii) an antibody-label conjugate, wherein said antibody-label conjugate binds to said two HLA antigens at a site other than said cross-reactive antibody wherein said kit is configured for use in the method of any of claims 1 to 7.

9. A kit according to claim 8, wherein said HLA antigens are B7 and B27, and further including B7 as a positive control.

10. A kit according to claims 8 or 9, wherein said label is an enzyme.

**Patentansprüche**

1. Verfahren zum Nachweisen der Gegenwart eines löslichen, ersten HLA-Antigens in einer Probe mit Antikörper, der mit dem ersten und einem zweiten HLA-Antigen kreuzreagiert, wobei das Verfahren Folgendes umfasst:

das Kombinieren von Aliquoten der Probe mit dem kreuzreagierenden Antikörper in einem ersten und einem zweiten Behälter in Abwesenheit bzw. Gegenwart von Anti-zweites HLA-Antigen, worin der kreuzreagierende Antikörper an die Oberfläche der Behälter gebunden ist,

das Kombinieren einer eigenen negativen Kontroliprobe, der das erste und das zweite HLA-Antigen fehlen, mit dem kreuzreagierenden Antikörper, und einer eigenen positiven Kontrollprobe, die das zweite HLA-Antigen umfasst, mit dem kreuzreagierenden Antikörper in Abwesenheit bzw. Gegenwart von Anti-zweites HLA-Antigen, worin die Gegenwart von Anti-zweites HLA-Antigen eine Verstärkung des Signals bewirkt;

die Zugabe eines Konjugats von Antikörper, der sich an einer anderen als der Bindungsstelle des kreuzreagierenden Antikörpers an gemeinsames Epitop des ersten und des zweiten HLA-Antigens bindet, sowie eines nachweisbaren Markers; und

die Bestimmung eines Signals vom nachweisbaren Marker im ersten und zweiten Behälter sowie in negativer und in positiver Kontrollprobe; worin Grenzwerte auf Basis der positiven und der negativen Kontrollprobe dazu dienen, um zu bestimmen, ob die Probe das erste HLA-Antigen umfasst.

**2.** Verfahren nach Anspruch 1, worin die negative und die positive Kontrollprobe in Verbindung mit der Bestimmung durchgeführt werden.

**3.** Verfahren zum Nachweis der Gegenwart von löslichem HLA-B27 in einer Probe mit kreuzreagierendem Antikörper, der mit HLA-B7 und -B27 kreuzreagiert, wobei das Verfahren Folgendes umfasst:

das Kombinieren von Aliquoten der Probe mit dem kreuzreagierenden Antikörper in einem ersten und einem zweiten Behälter in Gegenwart bzw. Abwesenheit von Anti-HLA-B7, worin der kreuzreagierende Antikörper an die Oberfläche der Behälter gebunden ist;

das Kombinieren einer eigenen negativen Kontrollprobe, der HLA-B27- und HLA-B7-Antigene fehlen, mit dem kreuzreagierenden Antikörper, und einer eigenen positiven Kontrollprobe, die HLA-B7-Antigen umfasst, mit dem kreuzreagierenden Antikörper in Abwesenheit bzw. Gegenwart von Anti-HLA-B7-Antigen, worin die Gegenwart von Anti-HLA-B7-Antigen eine Verstärkung des Signals bewirkt;

die Zugabe eines Konjugats von Antikörper, der sich an einer anderen als der Bindungsstelle des kreuzreaktiven Antikörpers an ein gemeinsames Epitop von HLA-B27 und HLA-B7 bindet, und eines nachweisbaren Markers; und

die Bestimmung eines Signals vom nachweisbaren Marker im ersten und zweiten Behälter sowie in der negativen und in der positiven Kontrollprobe;

worin Grenzwerte auf Basis der positiven und der negativen Kontrollprobe dazu dienen, um zu bestimmen, ob die Probe HLA-B27-Antigen umfasst.

**4.** Verfahren nach Anspruch 1 oder 3, worin die Probe eine Blutprobe ist.

**5.** Verfahren nach Anspruch 3, worin der kreuzreagierende Antikörper ein monoklonaler Antikörper mit einer stärkeren Affinität für HLA-B27 als für HLA-B7 ist.

**6.** Verfahren nach Anspruch 1 oder 3, worin eine Vielzahl von Proben unter Einsatz von Mikrotiterplatten getestet wird.

**7.** Verfahren nach Anspruch 1 oder 3, worin der Marker ein Enzym ist und das folgende zusätzliche Schritte umfasst:

die Zugabe von Enzymsubstrat zu den Behältern;
das Inkubieren für ausreichend lange Zeit, um ein Signal zu erzeugen und die Enzymreaktion zu beenden.

**8.** Set, das Folgendes umfasst: (i) eine Vielzahl von Behältern, die mit Antikörper beschichtet sind, der mit zwei HLA-Antigenen kreuzreaktiv ist; (ii) Antikörper, der für nur eines der HLA-Antigene spezifisch ist; (iii) ein Antikörper-Marker-Konjugat, worin sich das Antikörper-Marker-Konjugat an einer anderen Stelle als der kreuzreaktive Antikörper an die beiden HLA-Antigene bindet, worin das Set zur Verwendung im Verfahren nach einem der Ansprüche 1 bis 7 konfiguriert ist.

**9.** Set nach Anspruch 8, worin die HLA-Antigene B7 und B27 sind und das weiters B7 als positive Kontrollprobe umfasst.

**10.** Set nach Anspruch 8 oder 9, worin der Marker ein Enzym ist.

**Revendications**

**1.** Méthode pour déterminer la présence d'un premier antigène HLA soluble dans un échantillon avec un anticorps qui réagit mutuellement avec ledit premier et un second antigène HLA, ladite méthode comprenant :

la combinaison d'aliquotes dudit échantillon avec ledit anticorps réagissant mutuellement dans des premier et second conteneurs en l'absence et en présence de l'anti-second antigène HLA, respectivement, où ledit anticorps réagissant mutuellement et lié à la surface desdits conteneurs ;

la combinaison d'un contrôle négatif séparé manquant desdits premier et second antigènes HLA avec ledit anticorps réagissant mutuellement et d'un contrôle positif séparé comprenant ledit second antigène HLA avec ledit anticorps réagissant mutuellement en l'absence et en présence de l'anti-second antigène HLA, respectivement, où la présence de l'anti-second antigène HLA agit pour améliorer le signal ;

l'addition d'un conjugué de l'anticorps qui se lie à un épitope commun desdits premier et second antigènes HLA ailleurs qu'au site de liaison dudit corps réagissant mutuellement et un marqueur détectable ; et

la détermination d'un signal dudit marqueur détectable dans lesdites premier et second conteneurs et lesdits contrôles négatif et positif ; où des valeurs de coupure basées sur lesdits contrôles positif et négatif sont utilisées pour déterminer si ledit échantillon comprend ledit premier antigène HLA.

2. Méthode selon la revendication 1, où les contrôles négatif et positif sont accomplis en conjonction avec ladite détermination.

3. Méthode pour déterminer la présence de HLA-B27 soluble dans un échantillon avec un anticorps réagissant mutuellement, qui réagit mutuellement avec HLA-B7 et - B27, ladite méthode comprenant :

la combinaison d'aliquotes dudit échantillon avec ledit anticorps réagissant mutuellement dans des premier et second conteneurs en présence et en l'absence de anti-HLA-B7 respectivement, où ledit anticorps réagissant mutuellement est lié à la surface desdits conteneurs ;

la combinaison d'un contrôle négatif séparé manquant des antigènes HLA-B27 et HLA-B7 avec ledit anticorps réagissant mutuellement et un contrôle positif séparé comprenant l'antigène HLA-B7 avec ledit anticorps réagissant mutuellement en l'absence et en présence de l'anti-antigène HLA-B7, respectivement, où la présence de l'anti-antigène HLA-B7 agit pour améliorer le signal ;

l'addition d'un conjugué de l'anticorps qui se lie à un épitope commun de HLA-B7 et HLA-B27 ailleurs qu'au site de liaison dudit anticorps réagissant mutuellement et d'un marqueur détectable ; et

la détermination d'un signal dudit marqueur détectable dans lesdits premier et second conteneurs et lesdits contrôles négatif et positif ;

où des valeurs de coupure basées sur lesdits contrôles positif et négatif sont utilisées pour déterminer si ledit échantillon comprend l'antigène HLA-B27.

4. Méthode selon la revendication 1 ou 3, où ledit échantillon est un échantillon de sang.

5. Méthode selon la revendication 3, où ledit anticorps réagissant mutuellement est un anticorps monoclonal ayant une affinité plus grande pour HLA-B27 que pour HLA-B7.

6. Méthode selon les revendications 1 ou 3, où un certain nombre d'échantillons sont testés en utilisant des plaques de microtitration.

7. Méthode selon les revendications 1 ou 3, où ledit marquer est une enzyme et comprenant les étapes additionnelles de :

ajouter un substrat d'enzyme auxdits conteneurs ;

soumettre à incubation pendant un temps suffisant pour produire un signal et arrêter la réaction de l'enzyme.

8. Kit comprenant (i) un certain nombre de conteneurs enduits d'un anticorps réagissant mutuellement avec deux antigènes HLA, (ii) un anticorps spécifique d'un seul desdits antigènes HLA, (iii) un conjugué anticorps-marqueur, où ledit conjugué anticorps-marqueur se lie auxdits deux antigènes HLA en un site autre que ledit anticorps réagissant mutuellement, où ledit kit est configuré pour une utilisation dans la méthode selon l'une quelconque des revendications 1 à 7.

9. Kit selon la revendication 8, où les antigènes HLA sont B7 et B27, et comprenant de plus B7 comme contrôle positif.

10. Kit selon les revendications 8 ou 9, où ledit marqueur est une enzyme.